# EUROPEAN PATENT SPECIFICATION

(11) **EP 0 482 276 B1**
(45) Date of publication and mention of the grant of the patent: **03.01.1996**
(21) Application number: 90870199.8
(22) Date of filing: 22.10.1990
(51) Int. Cl.: C07C 15/40, C07C 5/333, B01J 37/16

(54) **Process for the catalytic dehydrogenation of alkylaromatic hydrocarbons**
Verfahren zur katalytischen Dehydrierung von alkylaromatischen Kohlenwasserstoffen
Procédé de déshydrogénation catalytique d'hydrocarbures alkylaromatiques

(43) Date of publication of application: 29.04.1992
(73) Proprietor: FINA RESEARCH S.A., B-7181 Feluy (BE)
(72) Inventor: Debras, Guy, B-6210 Les-Bons-Villers (BE); Grootjans, Jacques, B-3061 Leefdaal (BE); Bodart, Philippe, B-4480 Clermont-sous-Huy (BE); Delorme, Luc, B-1410 Waterloo (BE)

(56) References cited:
- EP-A- 0 397 637
- FR-A- 827 068
- GB-A- 840 082
- US-A- 3 118 007
- US-A- 4 607 129
- US-A- 4 644 089

## Description

The present invention relates to a process for the catalytic dehydrogenation of hydrocarbons. Particularly the present invention relates to the dehydrogenation of alkylaromatic hydrocarbons to produce the corresponding alkenylaromatic hydrocarbons. More particularly the process of the invention uses catalytic systems of the redox type.

Catalytic dehydrogenation of hydrocarbons has been carried out for many years and constitutes an important catalytic process in view of the increasing demand in dehydrogenated products which may be valorized under the most various forms such as high octane gasolines, plastic materials and synthetic rubbers.

In the field of dehydrogenation of alkylaromatic hydrocarbons, known processes include thermal dehydrogenation, catalytic dehydrogenation in the presence of an inert diluent such as steam, and oxidative dehydrogenation, the latter involving the injection of molecular oxygen in the reaction medium. However, although the oxidative dehydrogenation may have some advantages regarding reaction yield and selectivity of the desired product, it is also well known that the presence of molecular oxygen in the reaction medium leads to the formation of indesirable oxidation products such as aldehydes.

In view to partially obviate these drawbacks, it has often been proposed to use very specific catalysts having a particular selectivity towards oxidative dehydrogenation of certain hydrocarbons whether or not of the alkylaromatic type.

In this respect, it has already been proposed in US patent 4,777,319 to Kung et al. to use vanadates or mobybdates of metals for the selective dehydrogenation of paraffinic hydrocarbons having from 3 to 6 carbon atoms. However, the dehydrogenation reaction is necessarily carried out in the presence of molecular oxygen, which displaces the thermodynamic equilibrium but leads to the formation of secondary oxidation products.

It has also been proposed in US 4,742,180 to Gaffney to use supported catalysts, the support being essentially an oxide of praseodymium on which there is deposited an alkaline metal having a dehydrogenating action. However, the availability of praseodymium oxide for the production of huge amounts of alkenylaromatic hydrocarbons has to be taken into account. Moreover, it has to be noted that no significant result is indicated for the dehydrogenation of alkylaromatic hydrocarbons. Oganowski has also proposed to use a catalyst of the Mg₃(VO₄)₂ type for the dehydrogenation of ethylbenzene, but the reaction necessarily occurs in the presence of a gas containing molecular oxygen. Moreover, it is known that oxides of the V₂O₅ type lead to reaction of complete combustion when used in dehydrogenation reaction of hydrocarbons carried out without bringing in molecular oxygen.

US-A-3118007 discloses a process for the dehydrogenation of gaseous hydrocarbon with iron oxide containing catalysts in a fluidised bed, which comprises forming water with the oxygen derived from the iron oxide, said iron oxide being introduced in a high state of oxidation and being removed in a lower state of oxidation, substantially no free iron being formed, then reoxidising the catalyst and continuously recycling it.

GB-A-840082 discloses a process for the dehydrogenation of mono-olefins into diolefins by contacting them with an active contact material comprising one or more oxides of iron, cobalt or nickel in a high oxidation state at a temperature from 260°C to 540°C, regenerating the material by re-oxidation and recycling it.

It also discloses an embodiment wherein the regenerated material prior to being returned to contact the mono-olefinic feed is first contacted with a non-oxidizing gas to remove excessively loosely held oxygen from the freshly regenerated material.

The Applicant has proposed earlier (see EP-A-397 637) a process for the dehydrogenation of alkylaromatic hydrocarbons in the presence of a redox catalytic system. However, the process, although providing yields sometimes higher than 90 %, did not have a sufficient initial selectivity. Thus, in actual operations, alkylaromatics losses were excessive. There is thus a need in the art for a process that would have a higher selectivity.

An object of the present invention is to provide an improved process for the catalytic dehydrogenation of alkylaromatic hydrocarbons in the presence of a redox catalytic system.

Another object of the present invention is to provide an improved process for the catalytic dehydrogenation of alkylaromatic hydrocarbons in the absence of molecular oxygen.

A further object of the present invention is to provide an improved process for the catalytic dehydrogenation of alkylaromatic hydrocarbons in the presence of a redox catalytic system, whether or not supported, and of which one or more oxidation stages show a dehydrogenating activity.

A further object of the present invention is to provide a continuous process of improved selectivity for the dehydrogenation of alkylaromatic hydrocarbons in the presence of a redox catalytic system.

The present invention provides for a process for the catalytic dehydrogenation of alkylaromatic hydrocarbons into corresponding alkenylaromatic hydrocarbons which comprises :
- partially reducing catalyst consisting of at least one reducible oxide of a metal (I) selected from the group consisting of V, Cr, Mn, Pb, Bi, Mo, U and Sn, supported on a material selected from clays, zeolitic materials of the metallo-silicate or metallo-alumino-phosphate type, and oxides of a metal (II) selected from Ti, Zr, Zn, Th, Mg, Ca, Ba, Si, Al and mixtures thereof, said catalyst having a dehydrogenation activity when said metal (I) has a valency such that it is not in its most reduced state, with a reducing agent,
- contacting the hydrocarbon feedstock to be dehydrogenated under dehydrogenation conditions, in the absence of any gas containing molecular oxygen, with the partially reduced catalyst,
- recovering the dehydrogenated hydrocarbons,
- regenerating the used catalyst,
- contacting the regenerated catalyst with fresh hydrocarbon feedstock to be dehydrogenated.

The Applicant has now found that the selectivity of the catalyst is greatly increased by a partial reduction thereof prior to contacting it with hydrocarbon feedstock to be dehydrogenated, this fact constituting an important advantage over the prior processes.

Whilst not wishing to be bound by a theory, it would appear that the initial activity of the catalyst is excessive; significant amounts of carbon dioxide have in some instances been detected, leading to believe that alkylaromatic hydrocarbons are being lost for the economics of the process.

Further, oxygenated derivatives of alkylaromatics are formed, which impart a dark brown colour to the product of the dehydrogenation reaction and are very difficult to separate from the alkenylaromatic hydrocarbons.

The preliminary partial reduction of the catalyst is preferably carried out with a reducing agent whose oxidized form can be easily separated and does not impart a colour to the alkenylaromatic hydrocarbons whilst no alkylaromatics are converted. It is generally convenient that the pre-reducing agent should be a weaker reducing agent in the pre-reduction conditions than alkylaromatics in the dehydrogenation conditions. As suitable pre-reducing agents, there can be cited hydrogen, carbon monoxide, methane, ethane, propane, butane, and mixtures thereof, such as LPG (liquid petroleum gas). The reducing agents may be used either alone or in admixture with one or more inert gases.

According to a preferred embodiment, said partial reduction is preferably carried out with carbon monoxide, at a temperature comprised between 200 and about 800 °C, preferably between 300 and 550°C, and at a pressure comprised between 0.001 and 1 MPa, preferably about equal to atmospheric pressure. In a fixed-bed system, the contact time is preferably determined by monitoring the carbon dioxide in the outlet gases : there is little advantage in pursuing when the carbon dioxide yield is becoming less than 5 mol %, more particularly less than 1 mol %, of the carbon monoxide. In a fluidised-bed system, the CO/metal(I) molar ratio is set at a value greater than 0.2, preferably higher than 0.4, and adjusted if necessary.

According to the process of the invention, the feedstock of alkylaromatic hydrocarbons to be dehydrogenated is contacted with a catalyst which has to fulfil several conditions. The catalyst comprises at least a reducible oxide of a metal selected from V, Cr, Mn, Bi, Mo, U and Sn; reducible oxides as used herein mean the hereabove metal oxides which are reduced by contact with hydrocarbons, when operating under dehydrogenation conditions. Moreover, the metal oxide used has to have a dehydrogenating action under the reaction conditions.

Further, the Applicant has found that it is advantageous that these oxides be deposited on a support. By way of examples of suitable supports, it may be cited the oxides of metals selected from Ti, Zr, Zn, Mg, Th, Si, Ca, Ba and Al, as well as clays and zeolitic materials of the metallo-silicate or metallo-alumino-phosphate type. Within the zeolitic materials, it may be cited the alumino-silicates, the borosilicates, silico-alumino-phosphates and other analogues. Preferred supports are magnesium oxide, silicon oxide, zinc oxide, and mixtures thereof.

The supported catalysts used in the process of the invention may also comprise promoters such as alkali or alkaline-earth metals.

The supported catalyst may be prepared according to usual methods such as absorption, precipitation or still impregnation.

Among the various catalysts which may be used in the process of the invention, the Applicant has found that favourable results are obtained with catalysts of the vanadium oxide type deposited on a support comprising magnesium oxide and silicon oxide.

The contact between the feedstock to be dehydrogenated and the catalyst may be performed according to different ways; for instance the catalyst particles may be used in a fixed bed, or they may be used in a fluidized bed reactor wherein said particles are circulated and thereafter recovered, what implies a suitable distribution of the particles sizes.

According to an embodiment of the process of the present invention the catalyst is placed in a fixed bed and contacted with the feedstock of alkylaromatic hydrocarbons to be dehydrogenated, in the absence of a molecular oxygen containing gas, at a temperature comprised between 300 and 800°C, in order to maintain the feedstock in the vapor phase. When the activity of the catalyst is reduced over the limits which are currently acceptable, generally a reduction of 10 % to 30 % of the conversion, the feedstock is no longer contacted with the catalyst, and an air stream is passed over the catalyst bed at a temperature of from 200°C to 1000°C in order to regenerate said catalyst under mild conditions. This regeneration comprises at least a mild oxidation of the catalyst, and must include a subsequent partial reduction of the catalyst, as described above. When the catalyst is regenerated the feedstock is again contacted with the catalyst.

According to a preferred embodiment of the present invention, the catalyst particles, whose sizes are comprised between 0.02 and 0.3 mm, the catalyst being taken in its oxidized form, are circulating in the dehydrogenation reaction zone, and contacted with the feedstock to be dehydrogenated, in the absence of an oxygen containing gas, at a temperature comprised between 300 and 800°C in order to maintain the feedstock in the vapor phase. The gas pressure at the outlet of the reactor is generally comprised between 10⁵ and 1.3 10⁵ Pa while residence time of the feedstock in the reactor is of 0.5 to 15 seconds, while the residence time of the catalyst is comprised between 0.5 second and 5 minutes; the upper limit of the residence time of the catalyst depends obviously on its activity. According to this preferred embodiment, the dehydrogenation reaction and the transportation of the catalyst to the regenerator are more particularly carried out in a fluidized bed reactor.

The catalyst in the reactor effluent is separated from the hydrocarbon effluent by suitable means. The separated catalyst is a reduced catalyst because it is in a lower oxidation state than that of the fresh catalyst which enters the reaction zone. The separated catalyst is sent to a regeneration zone where it is regenerated with gaseous stream containing molecular oxygen. This regeneration comprises at least a mild oxidation of the catalyst. The temperature in the regeneration zone is most often maintained between about 200 and about 1000°C; the residence time of the catalyst in said zone is of at least about 5 seconds, while that of the gas containing molecular oxygen is usually of about 1 to 30 seconds. The amount of gas together with the oxygen concentration must be sufficient to reoxidize the catalyst in its initial form. The catalyst must then be partially reduced, as described hereabove, prior to contacting it with fresh alkylaromatic feedstock to be dehydrogenated. If this embodiment is used for the regeneration of the catalyst, then the dehydrogenation process may be carried out continuously, while it was not the case with the previous embodiment.

Among the various catalysts that may be used, the Applicant has found that favourable results are obtained, more particularly in the fluidised bed embodiment, with catalysts of the MgO - SiO₂ - V₂O₅ type which may also comprise promoters such as alkali or alkaline-earth metals. These catalysts comprise 40-60 wt % (preferably about 50 %) MgO, 20-40 wt % (preferably about 30 %) SiO₂, 10-30 wt % (preferably about 15 %) V₂O₅, and minor amounts of promoters (typically about 0,5 wt % CaO and about 1,5 wt % Na₂O) and of other impurities. They can be prepared by spray-drying of an MgO/SiO₂ suspension, followed by impregnation with an ammonium vanadate solution preferably also with a further amount of SiO₂ as silicasol, evaporation and calcination at a temperature of from 500°C to 800°C. Simultaneous spray-drying of an MgO/SiO₂ suspension in an ammonium vanadate solution can also be used, followed by a calcination at 500-800°C.

The process of the invention is suitable for the dehydrogenation of alkylaromatic hydrocarbons into corresponding alkenylaromatic hydrocarbons. Particularly, the process of the invention is applied to the catalytic dehydrogenation of ethylbenzene and ethyltoluene into styrene or vinyltoluene respectively. The process is generally carried out at a temperature comprised between 350 and 800°C and preferably between 400 and 650°C, at a pressure comprised between 0.001 and 1 MPa, and at a hourly space velocity comprised between about 0.01 and 20.0 kg of hydrocarbon per hour and per kg of catalyst, preferably between 1 and 10.

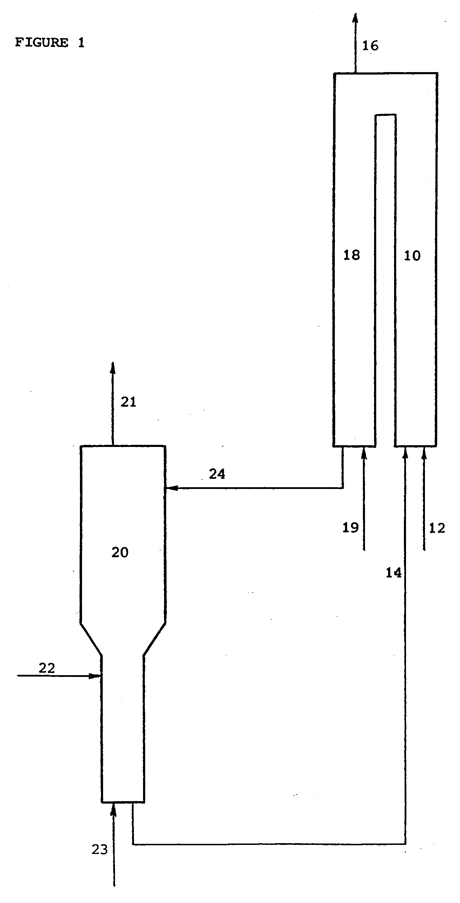

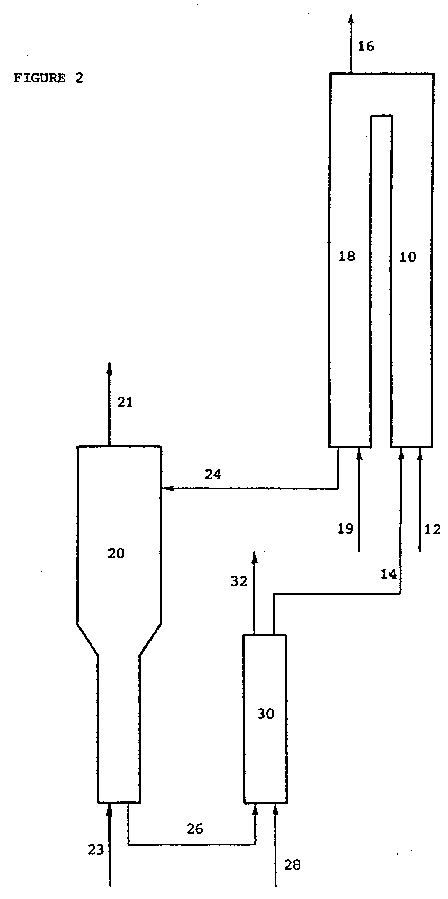

The preferred embodiment of the invention is also described by way of the drawings according to which Figures 1 and 2 represent schematic diagrams of the reaction zone and the regeneration zone of the catalyst in two different embodiments of continuous processes according to the invention.

Referring now to Figure 1, the feedstock of hydrocarbons to be dehydrogenated enters the dehydrogenation zone 10 through pipe 12 while the catalyst under its partially reduced form enters through pipe 14. The reduced catalyst is separated in the area 18 of the reactor, using e.g. a nitrogen or steam flow entering through pipe 19. The dehydrogenated feed is withdrawn through pipe 16, while the reduced catalyst is collected into pipe 24 and transported to the top of the regeneration reactor 20. Gas containing molecular oxygen is introduced into reactor 20 through pipe 22 while the pre-reducing agent is introduced through pipe 23. The regenerated catalyst is recovered in the outlet pipe 14 and recycled into the dehydrogenation reactor 10. Pipe 21 is the exit pipe for gases from reactor 20.

Figure 2 represents another embodiment of the invention, wherein the dehydrogenation is as described for Figure 1, while the regeneration occurs in two different reactors. The reduced catalyst is transported through pipe 24 to the top of the oxidation reactor 20. Gas containing molecular oxygen is introduced into reactor 20 through pipe 23 and exits through pipe 21, while the oxidized catalyst is recovered in the outlet pipe 26 and transported to the pre-reduction reactor 30. The pre-reducing agent is introduced through pipe 28 into reactor 30 and exits through pipe 32. The pre-reduced catalyst is recovered in the outlet pipe 14 and recycled into the dehydrogenation reactor 10.

The process of the present invention shows many advantages with respect to the usual processes and particularly it enables to avoid drastically the formation of oxygenated products, due to the fact that the presence of molecular oxygen is excluded. Moreover, hydrogen is eliminated upon its formation, which enables to displace the equilibrium of the reaction and to run at less high temperatures.

Therefore, it is no more necessary to use diluents such as inert gases to displace the equilibrium; however the process of the invention may also be carried out in the presence of usual diluents.

Another non-negligible advantage of the process of the invention resides in the fact that it allows to work at a less high temperature than with the prior processes.

A substantial increase of the selectivity is also noted. Further, it has been found that it was no more necessary to work under vacuum.

The process of the invention will be better illustrated by way of the following examples.

### Examples

### Preparation of the catalyst

Technical grade magnesium oxide (98 wt % MgO containing 0.8 wt % CaO, 0.3 wt % SiO₂ and 0.2 wt % Fe₂O₃ as major impurities) was used, under the form of particles (99 % of which had a size below 0.04 mm) having a specific area of 50.9 m²/g.

4.5 kg of said oxide and 3.7 kg of silicasol (containing 40.5 wt % SiO₂) were suspended in water. The suspension was spray-dried in a container kept at a temperature such that a powder was obtained having at least 70 wt % of particles between 0.04 and 0.15 mm.

The powder fraction between 0.04 and 0.15 mm was separated by sieving.

250 g of said fraction were suspended in 750 ml water. The suspension was heated to a temperature of 90°C for 1 hour. To the hot suspension, 77.2 g silicasol (40.5 wt % SiO₂) were added, then a 90°C solution of 71.8 g ammonium metavanadate in 1400 ml water.

The resulting mixture was introduced into a rotating flask kept in an oil bath and homogenised at 90°C during 15 minutes. The temperature of the oil bath was then raised to 120°C to dry the mixture maintained at that temperature and under slight nitrogen flushing for 18 hours.

The powder then obtained was sieved to retain particles between 0.04 and 0.15 mm and placed in an oven. The oven temperature was raised at a rate of 60°C/hour up to 600°C and kept at that temperature for 4 hours.

A catalyst was obtained, which was sieved to retain the particles between 0.04 and 0.15 mm. It contained 16 wt % of vanadium (expressed as V₂O₅), 30 % wt % of silicon (expressed as SiO₂), 1.5 wt % of sodium (expressed as Na₂O), and 0.45 wt % of calcium (expressed as CaO).

The catalyst had the following physical properties :

| | |
|---|---|
| - apparent packing density (ASTM D-4164-82) | 0.69 g/ml |
| - apparent bulk density (UOP standard method M.294-54T) | 0.58 g/ml |
| - surface area by BET technique (N₂ adsorption) | |
| - calcined at 500°C in air | 88 m²/g |
| - calcined at 120°C in vacuum | 53 m²/g |
| - volume of pores < 100 nm (nitrogen desorption) | 0.27 ml/g |
| - attrition loss (air jet velocity of 7.07 l/min under standard conditions) | |
| - from 0 to 5 hours | about 3.4 wt % |
| - from 5 to 25 hours | about 1.8 wt % |

### Examples 1 to 3, and Comparative Example A

### A. Experimental setup

A pulse microreactor was loaded with 500 mg catalyst (as hereabove obtained), heated at 500°C, using helium under atmospheric pressure as carrier gas.

### B. Catalyst pre-reduction

The catalyst was pre-reduced by passing thereon 400 pulses of 0,5 ml carbon monoxide each (Example 1) or 25 pulses of 5 ml carbon monoxide each (Example 2) or 50 pulses of 5 ml carbon monoxide each (Example 3), the gas volumes being measured at 0°C and under atmospheric pressure. No catalyst pre-reduction was used for Comparative Example A.

### C. Dehydrogenation conditions

10 pulses of ethylbenzene were passed over the catalyst, the amount of ethylbenzene being of 3.6 mg per pulse, and the contact time being of about 1.2 s.

### D. Catalyst reoxidation

In Example 2 and Comparative Example A, the reduced catalyst was oxidized by passing thereon 10 pulses of 5 ml air each (the volumes being measured aut 25°C and under atmospheric pressure.

### E. Results

Table 1 summarizes the average results obtained during the third to seventh pulses of ethylbenzene (EB), expressed as follows :
C = conversion of ethylbenzene = mol % of EB feed
S = styrene selectivity = mol % of converted EB

**Table 1**

| Example | Conditions | C | S |
|---|---|---|---|
| 1 | pre-reduction | 58.7 | 98.7 |
| A | first run | 94.3 | 96.3 |
| | second run after reoxidation | 93.9 | 96.6 |
| 2 | first run after pre-reduction | 62.4 | 97.9 |
| | second run after regeneration | 59.6 | 98.9 |
| | third run after regeneration | 69.4 | 99.0 |
| | fourth run after regeneration | 64.1 | 99.0 |
| 3 | pre-reduction | 62.3 | 98.9 |

These examples show that the selectivity reached with the process of the invention is higher than with previous ones. Whilst not whishing to be bound by a theory, this is probably due to an excessive oxidation of ethylbenzene, leading to carbon dioxide; the amount of carbon dioxide, expressed as mol % of the ethylbenzene feed, was determined in the second run of example 2 and of comparative example A, as shown in Table 2 for each pulse.

**Table 2**

| Pulse | Example 2 (run 2) | | | Comparative Example A (run 2) | | |
|---|---|---|---|---|---|---|
| | C | S | CO₂ | C | S | CO₂ |
| 1 | 95,2 | 97,9 | 3,1 | 99,9 | 67,0 | 200 |
| 2 | 84,5 | 98,5 | 1,7 | 99,7 | 89,6 | 57 |
| 3 | 70,3 | 98,6 | 1,2 | 99,1 | 94,2 | 29 |
| 4 | 62,3 | 98,6 | 1,0 | 97,9 | 95,9 | 19 |
| 5 | 56,6 | 98,7 | 0,8 | 95,6 | 96,8 | 13 |
| 6 | 52,2 | 98,7 | 0,7 | 91,9 | 97,2 | 9,8 |
| 7 | 51,8 | 98,8 | 0,6 | 87,0 | 97,5 | 8,2 |
| 8 | 50,4 | 98,8 | 0,5 | 83,0 | 98,5 | 6,7 |
| 9 | 46,8 | 98,8 | 0,5 | 80,6 | 97,9 | 5,3 |
| 10 | 42,5 | 98,8 | 0,5 | 77,3 | 98,0 | 4,5 |

A comparison between Example 3 and the first run of Example 2 shows that increasing the pre-reduction with carbon monoxide has no detrimental effect on the catalyst.

The conversion is apparently lower, but this is not the case if one considers that the unconverted feed is recycled. Simple calculations show that a conversion of 98.35 mol % and a selectivity of 96.6 mol % were obtained under the conditions of Example 2 by assuming that the unreacted ethylbenzene was recycled in the next run (Table 3).

**Table 3**

| Run | Ethylbenzene | Converted | Styrene |
|---|---|---|---|
| 2.1 | 100 | 62.4 | 61.1 (experimental) |
| 2.2 | 37.6 | 22.4 | 22.1 (calculated) |
| 2.3 | 15.2 | 10.55 | 10.4 (calculated) |
| 2.4 | 4.65 | 3.0 | 3.0 (calculated) |
| Total | | 98.35 | 96.6 (calculated) |

### Examples 4 to 7 and Comparative Example B

A fluidized bed reactor was used, with a setup as described by Figure 1. The catalyst was prepared as hereabove described and calcined at 550°C immediately before use. The experimental conditions and the results obtained are set out in Table 4.

**Table 4**

| | Fig. 1 | Ex. 4 | Ex. 5 | Ex. 6 | Ex. 7 | Comp.B |
|---|---|---|---|---|---|---|
| Temperature (°C) | | | | | | |
| Reactor | 10 | 500 | 500 | 500 | 500 | 500 |
| Stripper | 18 | 500 | 500 | 500 | 500 | 500 |
| Regenerator | 20 | 475 | 475 | 500 | 475 | 500 |
| Catalyst return | 14 | 500 | 500 | 500 | 500 | 520 |
| Feed entrance | 12 | 539 | 424 | 528 | 524 | 370 |

| Flow rates (l/h) | | | | | | |
|---|---|---|---|---|---|---|
| N₂ at stripper | 19 | 33 | 33 | 33 | 33 | 52 |
| Air at regenerator | 22 | 135 | 135 | 135 | 135 | 369* |
| N₂ at regenerator | 23 | 330 | 330 | 330 | 380 | 480 |
| CO at regenerator | 23 | 108 | 108 | 108 | 71.4 | 0 |
| Catalyst (g/min) | 12 | 70 | 70 | 70 | 70 | 50 |
| Ethylbenzene (ml/h) | 12 | 100 | 200 | 100 | 100 | 150 |
| Contact time (s) | 10 | 6.7 | 3.7 | 6.5 | 6.6 | 5.2 |

| Results (mol %) | | | | | | |
|---|---|---|---|---|---|---|
| EB conversion | | 60.9 | 47.2 | 61.4 | 63.8 | 63.0 |
| Styrene selectivity | | 99.6 | 96.1 | 94.4 | 92.6 | 83.8 |

| | | | | | | |
|---|---|---|---|---|---|---|
| * at pipe 23 | | | | | | |

These Examples show that the styrene selectivity in a continuous process according to the invention is greatly improved over the prior art (represented by Comparative Example B).

## Claims

1. Process for the catalytic dehydrogenation of alkylaromatic hydrocarbons into the corresponding alkenylaromatic hydrocarbons, characterized in that it comprises the steps of
- partially reducing a catalyst consisting of at least one reducible oxide of a metal (I) selected from the group consisting of V, Cr, Mn, Pb, Bi, Mo, U and Sn, supported on a material selected from clays, zeolitic materials of the metallo-silicate or metallo-alumino-phosphate type, and oxides of a metal (II) selected from Ti, Zr, Zn, Th, Mg, Ca, Ba, Si, Al and mixtures thereof, said catalyst having a dehydrogenation activity when said metal (I) has a valency such that it is not in its most reduced state,
- contacting the hydrocarbon feedstock to be dehydrogenated under dehydrogenation conditions, in the absence of any gas containing molecular oxygen, with the partially reduced catalyst,
- recovering the dehydrogenated hydrocarbons,
- regenerating the used catalyst, and
- contacting the regenerated catalyst with fresh hydrocarbon feedstock to be dehydrogenated.

2. Process according to claim 1, wherein a catalyst comprising of at least a vanadium oxide is used.

3. Process according to either of claims 1 and 2, wherein the catalyst is supported on a material selected from the group consisting of magnesium oxide, silicon oxide, zinc oxide and mixtures thereof.

4. Process according to either of claims 1 and 2, wherein the catalyst is supported on a zeolitic material selected from alumino-silicates, borosilicates and silico-alumino-phosphates.

5. Process according to any one of claims 1 to 4, characterized in that the dehydrogenation of alkylaromatic hydrocarbons and the recovery of the catalyst are carried out in a fluidized bed reactor.

6. Process according to any one of claims 1 to 4, characterized in that the supported catalyst is regenerated by passing on the catalyst bed a gaseous flow containing molecular oxygen at a temperature of from 200 to 1000°C, followed by a partial reduction of the oxidized catalyst.

7. Process according to any one of claims 1 to 5, wherein the catalyst recovered from the dehydrogenation reactor is regenerated in a second reactor of the fluidized bed type wherein it is reoxidized by a gaseous flow containing molecular oxygen at a temperature of from 200 to 1000°C, and pre-reduced before recycling to the dehydrogenation reactor.

8. Process according to any one of claims 1 to 5, wherein the catalyst recovered from the dehydrogenation reactor is sent into a second reactor of the fluidised bed wherein it is reoxidized by a gaseous flow containing molecular oxygen at a temperature of from 200 to 1000°C, then into a third reactor where it is pre-reduced before recycling to the dehydrogenation reactor.

9. Process according to any one of claims 1 to 8, wherein the dehydrogenation reaction is carried out, in the absence of gas containing molecular oxygen, at a temperature of from 350 to 800°C, preferably between 400 and 650°C, at a pressure of from 0.001 to 1 MPa, and with an hourly spatial velocity comprised between 0.01 and 20 kg of hydrocarbon per hour and per kg of catalyst.

10. Process according to any one of claims 1 to 9, wherein the partial reduction is carried out with a pre-reducing agent selected from the group consisting of hydrogen, carbon monoxide, methane, ethane, propane, butane, and mixtures thereof, at a temperature of from 200 to 800°C and at a pressure of from 0.001 to 1 MPa.

11. Process according to any one of claims 1 to 10, wherein the partial reduction is carried out with carbon monoxide, at a temperature of from 300 to 550 °C and at a pressure about equal to atmospheric pressure.

## Patentansprüche

1. Verfahren zur katalytischen Dehydrierung von alkylaromatischen Kohlenwasserstoffen zu den entsprechenden alkenylaromatischen Kohlenwasserstoffen, dadurch gekennzeichnet, daß es die folgenden Schritte umfaßt:
- teilweises Reduzieren eines Katalysators, der aus mindestens einem reduzierbaren Oxid eines Metalls (I), das aus der Gruppe ausgewählt wird, die aus V, Cr, Mn, Pb, Bi, Mo, U und Sn besteht, und das von einem Material, ausgewählt aus Tonen, Zeolithmaterialien des Metallsilikat- oder Metallaluminiumphosphattyps getragen wird, und Oxiden eines Metalls (II), ausgewählt aus Ti, Zr, Zn, Th, Mg, Ca, Ba, Si, Al und Gemischen davon besteht, wobei der Katalysator eine Dehydrierungswirksamkeit aufweist, wenn das Metall (I) eine derartige Valenz aufweist, daß es sich nicht in seinem am stärksten reduzierten Zustand befindet,
- Inkontaktbringen des zu dehydrierenden Kohlenwasserstoffausgangsmaterials mit dem teilweise reduzierten Katalysator unter Dehydrierungsbedingungen in Abwesenheit von jeglichem Gas, das molekularen Sauerstoff enthält,
- Gewinnen der dehydrierten Kohlenwasserstoffe,
- Regenerieren des gebrauchten Katalysators, und
- Inkontaktbringen des regenerierten Katalysators mit frischem zu dehydrierenden Kohlenwasserstoffausgangsmaterial.

2. Verfahren nach Anspruch 1, wobei ein mindestens ein Vanadiumoxid umfassender Katalysator verwendet wird.

3. Verfahren nach einem der Ansprüche 1 und 2, wobei der Katalysator von einem Material getragen wird, das aus der Gruppe ausgewählt wird, die aus Magnesiumoxid, Siliziumoxid, Zinkoxid und deren Gemischen besteht.

4. Verfahren nach einem der Ansprüche 1 oder 2, wobei der Katalysator von einem Zeolithmaterial getragen wird, das aus Aluminiumsilikaten, Borsilikaten und Silizium-Aluminiumphophaten ausgewählt wird.

5. Verfahren nach einem der Ansprüche 1 bis 4, dadurch gekennzeichnet, daß die Dehydrierung der alkylaromatischen Kohlenwasserstoffe und die Gewinnung des Katalysators in einem Wirbelschichtreaktor durchgeführt wird.

6. Verfahren nach einem der Ansprüche 1 bis 4, dadurch gekennzeichnet, daß der getragene Katalysator dadurch regeneriert wird, daß ein molekularen Sauerstoff enthaltender Gasstrom bei einer Temperatur von 200 bis 1000 °C über das Katalysatorbett geleitet wird, gefolgt von einer teilweisen Reduktion des oxidierten Katalysators.

7. Verfahren nach einem der Ansprüche 1 bis 5, wobei der aus dem Dehydrierungsreaktor wiedergewonnene Katalysator in einem zweiten Reaktor vom Wirbelbettyp regeneriert wird, in dem er mit einem molekularen Sauerstoff enthaltenden Gasstrom bei einer Temperatur von 200 bis 1000 °C rückoxidiert wird, und vor Rückführung in den Dehydrierungsreaktor vorreduziert wird.

8. Verfahren nach einem der Ansprüche 1 bis 5, wobei der aus dem Dehydrierungsreaktor wiedergewonnene Katalysator in einen zweiten Reaktor vom Wirbelbettyp geschickt wird, in dem er mit einem molekularen Sauerstoff enthaltenden Gasstrom bei einer Temperatur von 200 bis 1000 °C rückoxidiert wird, und dann in einen dritten Reaktor geschickt wird, in dem er vor Rückführung in den Dehydrierungsreaktor vorreduziert wird.

9. Verfahren nach einem der Ansprüche 1 bis 8, wobei die Dehydrierungsreaktion in Abwesenheit eines molekularen Sauerstoff enthaltenden Gases bei einer Temperatur von 350 bis 800 °C, vorzugsweise zwischen 400 und 650 °C, bei einem Druck von 0,001 bis 1 MPa und mit einer Stunden-Raumgeschwindigkeit zwischen 0,01 und 20 kg Kohlenwasserstoff pro Stunde und pro kg Katalysator durchgeführt wird.

10. Verfahren nach einem der Ansprüche 1 bis 9, wobei die teilweise Reduktion mit einem Vorreduzierungsmittel, das aus der Gruppe ausgewählt wird, die aus Wasserstoff, Kohlenmonoxid, Methan, Ethan, Propan, Butan und Gemischen davon besteht, bei einer Temperatur von 200 bis 800 °C und bei einem Druck von 0,001 bis 1 MPa durchgeführt wird.

11. Verfahren nach einem der Ansprüche 1 bis 10, wobei die teilweise Reduktion mit Kohlenmonoxid bei einer Temperatur von 300 bis 550 °C und bei einem Druck durchgeführt wird, der in etwa dem Atmosphärendruck entspricht.

## Revendications

1. Procédé de déshydrogénation catalytique d'hydrocarbures alkylaromatiques en hydrocarbures alkénylaromatiques correspondants caractérisé en ce que :
- l'on réduit partiellement un catalyseur constitué par au moins un oxyde réductible d'un métal (I) choisi parmi le groupe comprenant V, Cr, Mn, Pb, Bi, Mo, U et Sn, supporté sur un matériau choisi parmi les argiles, les matériaux zéolitiques du type métallo-silicate ou métallo-alumino-phosphate et les oxydes de métal (II) choisi parmi Ti, Zr, Zn, Th, Mg, Ca, Ba, Si, Al et leurs mélanges, ce catalyseur ayant une activité déshydrogénante lorsque le métal (I) est pris à une valence telle qu'il ne soit pas dans son état réduit maximum,
- l'on met en contact la charge d'hydrocarbures à déshydrogéner dans des conditions de déshydrogénation, en l'absence d'un gaz contenant de l'oxygène moléculaire, avec le catalyseur partiellement réduit,
- l'on récupère les hydrocarbures déshydrogénés,
- l'on régénère le catalyseur ayant servi, et
- l'on met en contact le catalyseur régénéré avec la charge fraîche d'hydrocarbures à déshydrogéner.

2. Procédé selon la revendication 1, caractérisé en ce que l'on utilise un catalyseur constitué par au moins un oxyde de vanadium.

3. Procédé selon l'une quelconque des revendications 1 et 2, caractérisé en ce que le catalyseur est supporté sur un matériau choisi parmi le groupe constitué d'un oxyde de magnésium, d'un oxyde de silicium, d'un oxyde de zinc et de leurs mélanges.

4. Procédé selon l'une quelconque des revendications 1 et 2, caractérisé en ce que le catalyseur est supporté sur un matériau zéolitique choisi parmi les alumino silicates, les borosilicates et les silico alumino phosphates.

5. Procédé selon l'une quelconque des revendications 1 à 4, caractérisé de ce que l'on réalise la déshydrogénation des hydrocarbures alkylaromatiques et la récupération du catalyseur dans un réacteur à lit fluidisé.

6. Procédé selon l'une quelconque des revendications 1 à 4, caractérisé en ce que le catalyseur supporté est régénéré en faisant passer sur le lit catalytique un courant gazeux contenant de l'oxygène moléculaire à une température de 200 à 1000°C, suivi d'une réduction partielle du catalyseur oxydé.

7. Procédé selon l'une quelconque des revendications 1 à 5, caractérisé en ce que le catalyseur récupéré du réacteur de déshydrogénation est régénéré dans un second réacteur de type à lit fluidisé dans lequel il est réoxydé par un courant gazeux contenant de l'oxygène moléculaire à une température de 200 à 1000°C, et pré-réduit avant recyclage dans le réacteur de déshydrogénation.

8. Procédé selon l'une quelconque des revendications 1 à 5, caractérisé en ce que le catalyseur récupéré du réacteur de déshydrogénation est envoyé dans un second réacteur à lit fluidisé dans lequel il est réoxydé par un courant gazeux contenant de l'oxygène moléculaire à une température de 200 à 1000°C, et ensuite envoyé dans un troisième réacteur ou il est pré-réduit avant recyclage dans le réacteur de déshydrogénation.

9. Procédé selon l'une quelconque des revendications 1 à 8, caractérisé en ce que l'on effectue la réaction de déshydrogénation, en l'absence de gaz contenant de l'oxygène moléculaire, à une température comprise entre 350 et 800°C, de préférence entre 400 et 650°C, à une pression comprise entre 0,001 et 1 MPa et à une vitesse spatiale horaire comprise entre 0,01 et 20 kg d'hydrocarbure par heure et par kg de catalyseur.

10. Procédé selon l'une quelconque des revendications 1 à 9, caractérisé en ce que l'on effectue la réduction partielle avec un agent pré-réducteur choisi parmi le groupe constitué par l'hydrogène, le monoxyde de carbone, le méthane, l'éthane, le propane, le butane, et leurs mélanges, à une température comprise entre 200 et 800°C et à une pression comprise entre 0,001 et 1 MPa.

11. Procédé selon l'une quelconque des revendications 1 à 10, caractérisé en ce que l'on effectue la réduction partielle avec le monoxyde de carbone, à une température comprise entre 300 et 550°C et à une pression à peu près égale à la pression atmosphérique.
